# EUROPEAN PATENT SPECIFICATION

(11) **EP 2 845 589 B1**
(45) Date of publication and mention of the grant of the patent: **04.07.2018**
(21) Application number: 14183537.1
(22) Date of filing: 04.09.2014
(51) Int. Cl.: A61K 31/122, A61K 31/365, A61K 31/525, A61K 31/7048, A61K 33/06, A61K 36/19, A61K 36/28, A61P 29/00, A61P 25/02, A61P 25/06, A61P 25/04

(54) **Composition for the prevention and treatment of migraine or neuropathic pain**
Zusammensetzung zur Vorbeugung und Behandlung von Migräne oder neuropathischem Schmerz
Composition pour la prévention et le traitement de la migraine ou de la douleur neuropathique

(30) Priority: 06.09.2013 IT MI20131474
(43) Date of publication of application: 11.03.2015
(73) Proprietor: FB HEALTH S.P.A., 63100 Ascoli Piceno (IT)
(72) Inventor: Marchetti, Marco, 63074 San Benedetto del Tronto (IT); Scapagnini, Giovanni, 95131 Catania (IT); Porcaro, Piero, 82100 Benevento (IT); De Grossi Mazzorin, Federico, 00123 Roma (IT)
(74) Representative: Coppo, Alessandro

(56) References cited:
- WO-A1-02/09698
- WO-A1-03/007975
- WO-A1-2012/091685
- US-A1- 2006 263 449
- A. L. WENTZ ET AL: "A double-blind, randomized, placebo-controlled, single-dose study of the cyclooxygenase-2 inhibitor, GW406381, as a treatment for acute migraine", EUROPEAN JOURNAL OF NEUROLOGY, vol. 15, no. 4, 1 April 2008 (2008-04-01), pages 420-427, XP055114609, ISSN: 1351-5101, DOI: 10.1111/j.1468-1331.2008.02093.x
- HIDEMICHI SUYAMA ET AL: "Effect of etodolac, a COX-2 inhibitor, on neuropathic pain in a rat model", BRAIN RESEARCH, vol. 1010, no. 1-2, 1 June 2004 (2004-06-01), pages 144-150, XP055114610, ISSN: 0006-8993, DOI: 10.1016/j.brainres.2004.03.014
- C TASSORELLI ET AL: "Parthenolide is the component of tanacetum parthenium that inhibits nitroglycerin-induced Fos activation: studies in an animal model of migraine", CEPHALALGIA, vol. 25, no. 8, 17 March 2005 (2005-03-17) , pages 612-621, XP055114304, NO ISSN: 0333-1024, DOI: 10.1111/j.1468-2982.2005.00915.x
- "COX-2 inhibitors", EXPERT OPINION ON THERAPEUTIC PATENTS 20030901 GB, vol. 13, no. 9, 1 September 2003 (2003-09-01), pages 1465-1468, ISSN: 1354-3776

## Description

### FIELD OF THE INVENTION

The present invention relates to a composition for the prophylaxis or treatment of migraine or neuropathic pain.

The present invention origins in the field of dietary supplements and herbal drugs. Specifically, the present invention concerns with dietary supplements or herbal drugs for preventing or treating acute or chronic migraine or neuropathic pain.

### PRIOR ART

Migraine is a primary headache syndrome affecting around 16 to 20% of the world population. Two main types of migraine are known. Classic migraine also known as migraine with aura affects around 7 to 9% of the population. This type is characterized by one symptoms, such as visual, sensory, and motor symptoms which appear before the migraine attack. The other type of migraine, without aura, is more common and affects around of 14 to 16% of the population. In common migraine the headache usually is unilateral, pulsating with an intensity from moderate to severe which may last from few hour to three days.

Many patients have a readily diagnosed susceptibility to the development of migraine headaches for which known drug therapies attempt either to prevent or reduce the number of migraine headaches or mitigate their debilitating effects on the patient.

At present numerous well known active ingredients are used in the prophylaxis and palliation of migraine and its symptoms. These active ingredients include beta-adrenergic receptor blocking agents such as propranolol (Diamond, S. et al., Long-term study of propranolol in the treatment of migraine. Headache, 22:268-271, 1982), tricyclic antidepressants such as amitriptyline (Ziegler, D.K. et al., Migraine prophylaxis. A comparison of propanolol and amitriptyline. Arch. Neurol., 44:486-489, 1987), calcium antagonists such as flunarizine (Diamond, S. and Freitag, F.G. A double blind trial of flunarizine in migraine prophylaxis. In: New Advances in Headache Research: 2. 349-254, 1991, Clifford Rose, F. Ed. Smith-Gordon: London), divalproex sodium (Hering, R. and Kuritzky, A. Sodium valproate in the prophylactic treatment of migraine: A double-blind study versus placebo. Cephalalgia, 12:81-84, 1992), non-steroidal anti-inflammatory agents like naproxen sodium (Bellavance, AJ. and Meloche, J.P. A comparative study of naproxen sodium, pizotyline and placebo in migraine prophylaxis. Headache, 30:710-715, 1990), ergotamine, dihydroergotamine (Young, W.B. Appropriate use of ergotamine tartrate and dihydroergotamine in the treatment of migraine: current perspectives. Headache, 37(suppl.1):S42-S45, 1997), and serotonin-Id receptor agonists like sumatriptan (Cady, R.K. et al., Treatment of acute migraine with subcutaneous sumatriptan. JAMA, 265:2831-2835, 1991).

TASSORELLI ET AL: "Parthenolide is the component of tanacetum parthenium that inhibits nitroglycerin-induced Fos activation: studies in an animal model of migraine",CEPHALALGIA, vol. 25, no. 8, 17 March 2005 (2005-03-17), pages 612-621, discloses the anti-migraine activity of parthenolide. US2006/0263449 discloses that an extract of Andrographis paniculata containing 1-3 wt.% andrographolide is effective against neuralgia caused by ankylosing spondylitis. However none of the above active ingredients has proven to be satisfactory in achieving an effective management or prevention of migraine attacks. A similar situation is encountered with neuropathic pain.

Neuropathic pain is a pain determined by damage or disease that affects the somatosensory system. Neuropathic pain may be associated with abnormal sensations called dysesthesia, and pain produced by normally non-painful stimuli (allodynia). Neuropathic pain may have continuous and/or episodic (paroxysmal) components. Neuropathic pain usually manifests as burning or coldness, "pins and needles" sensations, numbness and itching. Nociceptive pain is more commonly described as aching.

Although multiple conditions may generate neuropathic pain, a common underlying mechanism is the presence of inflammation at the site of the affected nerve(s).This inflammatory response initiates a cascade of events resulting in increased local perfusion, increased capillary permeability, and the concentration and activation of innate immune cells at the site of tissue injury, irritation, or infection. The resultant neuro-inflammatory environment can cause the activation of microglia and astrocytes located in the spinal cord and brain, which appear to play a prominent role in nociception. Inflammation in any part of the human body is coupled with pain, due to the release of inflammatory mediators such as prostaglandin E2 (PGE2), the pro-inflammatory cytokinesTNF-α and IL-1β, and nerve growth factor (NGF).These mediators, produced by non-neural cells or immune cells, can stimulate nociception terminals in the peripheral tissue to increase pain sensitivity.

At present there is still a need for obtaining remedies suitable for preventing the development of migraine attacks and neuropathic pain and for alleviating or remedying the accompanying symptomatology.

It is a general object of the present invention to provide new agents which are useful tools for the management of migraine attacks and for treating or preventing neuropathic pain as well.

An additional object of the present invention resides in the provision of a composition for treating or preventing neuropathic pain or migraine.

It is also an object of the present invention the provision of naturally occurring active ingredients or substances for preventing or treating migraine or neuropathic pain.

### SUMMARY OF THE INVENTION

In accordance with certain aspects of the present invention, the inventors have discovered that the administration of a combination or mixture of selected terpenes and terpenoids is effective in reducing the symptomatology accompanying migraine attacks and/or neuropathic pain.

The inventors have also founds that the association of plant extracts containing selected terpenes, especially a sesquiterpene lactone, and selected terpenoids as defined in the appended claims is effective in the prophylaxis and/or treatment of migraine attacks and/or neuropathic pain.

In accordance with a first aspect the present invention thus provides a composition for use in the prophylaxis and/or treatment of migraine and or neuropathic pain comprising a sesquiterpene lactone and a terpenoid both in an effective amount and optionally an edible carrier wherein the sesquiterpene lactone is parthenolide and the plant terpenoid is andrographolide, neo-andrographolide, deoxyandrographolide and mixtures thereof.

In certain preferred embodiments the terpenes and terpenoids are of vegetal origins.

Typically, the terpenoid is a diterpenoid lactone.

In accordance with certain embodiments the composition of the invention contains plant extracts or portions or vegetal tissues of plants containing a) the sesquiterpene lactone parthenolide and b) a diterpenoid lactone selected from andrographolide, neo-andrographolide, deoxyandrographolide and mixtures thereof.

In some embodiments the plant extracts containing the sesquiterpene lactone and the diterpenoid lactone are in the form of dry vegetal extracts.

The combination of the sesquiterpene lactone parthenolide and a plant terpenoid selected from andrographolide, neo-andrographolide, deoxyandrographolide and mixtures thereof or of plant extracts containing such bioactive ingredients/components provides a synergistic analgesic effect on migraine attacks and on neuropathic pain.

Typically, the synergistic bioactive components/ingredients of the composition may be of vegetal origin.

In accordance with a second aspect the present invention provides a composition comprising plant extracts or vegetal portions of a plant containing a sesquiterpene lactone, specifically parthenolide, and a diterpenoid lactone selected from andrographolide, neo- andrographolide, deoxyandrographolide and mixtures thereof. The above composition is suitable for use in the prevention or treatment of a migraine attack and/or neuropathic pain.

In certain embodiments the plant terpenoid is andrographolide.

According to certain embodiments, the composition of the invention is a pharmaceutical composition, a phytomedicine, supplement or nutraceutical which may be introduced in the dietary regimen of a subject which is susceptible of migraine attacks or neuropathic pain.

In accordance with certain aspects of the invention a product or kit containing the sesquiterpene lactone parthenolide and a diterpenoid lactone selected from andrographolide, neo- andrographolide, deoxyandrographolide and mixtures thereof as a combined preparation for simultaneous, separate or sequential use in the prophylaxis or treatment of migraine or neuropathic pain is provided.

The inventors have also found for the first time that a diterpenoid lactone selected from andrographolide, neo-andrographolide, deoxyandrographolide, andrographolide especially, is active in the prophylaxis or treatment of migraine and/or neuropathic pain.

In accordance with certain aspects a diterpenoid lactone selected from andrographolide, neo-andrographolide, deoxyandrographolide and mixtures thereof is provided for use in the prophylaxis or treatment of migraine and/or neuropathic pain.

Certain characteristics and advantages of the present invention will become evident from the following drawings.

### BRIEF DESCRIPTION OF THE DRAWINGS

Fig.1 shows bar charts illustrating the synergistic effect of a composition containing parthenolide (PAR) and andrographolide (AND) on inflammatory cytokines secretion in cultured microglia, according to Example 3.
Figs. 2 shows graphical bars illustrating the lower DNA binding activity observed in microglial cells with LPS stimulation in presence of a combination of parthenolide (PAR) and andrographolide (AND) according to Example 4.
Fig.3 shows bar charts illustrating the time-dependent increase in Nrf2 protein expression in the nuclear extracts of Example 5, after treatment with parthenolide and andrographolide alone and in combination.

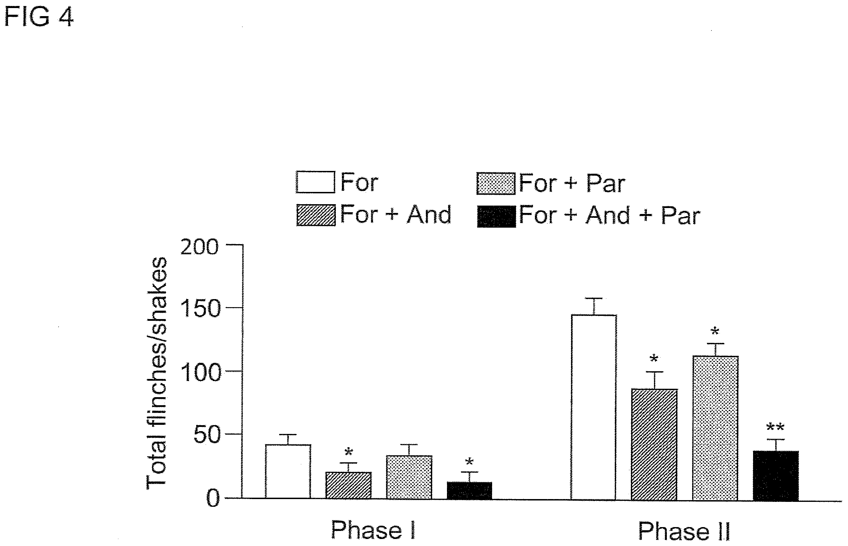

Fig.4 illustrates bar graphs attesting the synergistic effect of a treatment with a composition containing parthenolide (PAR) and andrographolide (AND) on hyperalgesia at the formalin test in rats according to Example 6.
Fig. 5 shows bar graph illustrating the synergistic effect of a treatment with a composition containing parthenolide (PAR) and andrographolide (AND) on nitroglycerin induced hyperalgesia at the formalin test in rats, according to Example 6.

### DETAILED DESCRIPTION OF THE INVENTION

In accordance with certain aspects of the invention, the inventors found that the administration of the sesquiterpene lactone parthenolide with a diterpenoid lactone selected from andrographolide, neo-andrographolide, deoxyandrographolide and mixtures thereof reduces the number of migraine attacks and the intensity of the correlated symptomatology and the neuropathic pain as well.

In accordance with a first aspect the present invention provides a composition for use in the prophylaxis and/or treatment of migraine and or neuropathic pain comprising a synergistic combination or mixture of a sesquiterpene lactone and a diterpenoid lactone and optionally an edible carrier, wherein the sesquiterpene lactone is parthenolide and the plant terpenoid is selected from andrographolide, neo-andrographolide, deoxyandrographolide and mixtures thereof.

One of the bioactive ingredients or components of the composition of the invention is the sesquiterpene lactone parthenolide.

Parthenolide is a sesquiterpene lactone which typically is extracted from *Tanacetum parthenium* a plant also known as feverfew, a member of the Asteracee family.

Conveniently, suitable sesquiterpene lactones, such as parthenolide, may be obtained by extraction from *Tanacetum parthenium.*

The extraction of the sesquiterpene lactone parthenolide from a plant part of *Tanacetum parthenium* may be carried out by conventional techniques for example by petrol extraction of the aerial parts of the plant as reported by Bohlman and Zdero in Phytochemestry 1982; 21:2543-49.

A suitable parthenolide may be obtained by extraction from leaves or flowers of plants naturally containing sesquiterpene lactones such as *Tanacetum parthenium.*

According to certain embodiments the sesquiterpene lactone parthenolide is in the form of a vegetal extract.

In certain embodiments one ingredient of the composition of the invention is a dry extract from *Tanacetum parthenium* also called Partenio, in particular an extract from its leaves. Typically the extract from *Tanacetum parthenium* contains the sesquiterpene lactone parthenolide as an active ingredient.

Typically the extraction of a sesquiterpene lactone such as parthenolide from a plant such as *Tanacetum parthenium* may be made using a polar organic solvent. In certain embodiments a suitable sesquiterpene lactone, parthenolide especially, is obtained by contacting a plant tissue for example from Tanacetum parthenium in a polar organic solvent for example selected from a C₁-C₃ alcohol, ether, acetonitrile, ethyl acetate, acetone or mixtures thereof. Ethanol is a preferred solvent due to its very low toxicity.

Typically, during extraction parts of a plant, typically leaves of *Tanacetum parthenium*, are placed in contact with a suitable polar organic solvent such as ethanol, and left to stand whereby allowing the extraction of siotable sesquiterpenes lactones such as parthenolide to take place. In alternative suitable sesquiterpenes may be extracted with a polar organic solvent in a Soxhlet apparatus or similar apparatus. The plant tissue useful for extraction may be fresh, dried or frozen and may be in comminuted form. Generally, the plant extract containing sesquiterpene lactones is separated from plant tissue and the solvent removed by conventional techniques. Following removal of the solvent the remaining basic extract containing sesquiterpene lactones may be purified with conventional techniques such as column chromatography, recrystallization or further solvent extraction. The remaining plant extract may be further extracted using the same organic solvent or alternative solvents.

The extract containing sesquiterpene lactone, parthenolide especially, may be utilized as primary material for processing into a finished pharmaceutical preparation. By way of an example, the extract may be mixed with a terpenoid of vegetal origin such as andrographolide or a plant extract containing andrographolide as active ingredient and with at least one physiologically acceptable excipient and/or vehicle for preparing a phytomedicament, supplement or pharmaceutical product.

In accordance with certain embodiments one of the active ingredient of the composition is a dry extract from *Andrographis Paniculata.* Typically, the extract from *Andrographis Paniculata* contains a diterpenoid lactone selected from andrographolide, neo-andrographolide, deoxy-andrographolide and mixtures thereof.

In certain embodiments, the sesquiterpene lactone such as parthenolide or a vegetal extract containing parthenolide may be present in the composition of the invention in an amount of 0,001 to 35% by weight, 0,01 to 20% by weight or 0,1 to 10% by weight.

A diterpenoid lactone selected from andrographolide, neo-andrographolide, deoxy-andrographolide and mixtures thereof or a vegetal extract, portion or tissue of a plant containing such plant terpenoid is a bioactive ingredient/component of the composition of the invention.

A suitable diterpenoid lactone may be of vegetal or synthetic origin.

In certain preferred embodiments the diterpenoid lactone is of vegetal origin, in particular obtained by extraction from a plant.

In certain embodiment the plant diterpenoid lactone is contained or extracted from *Andrographis paniculata,* a herbaceous plant in the family of Acanthaceae. In particular the leaves of as *Andrographis paniculata* are rich of the terpenoids of the composition of the invention.

In certain embodiments the diterpenoid lactone is in the form of a vegetal extract containing a diterpenoid lactone.

Generally, suitable terpenoids are the diterpenoid lactones: andrographolide (a), neo-andrographolide (b), deoxyandrographolide (c) having the following formulae

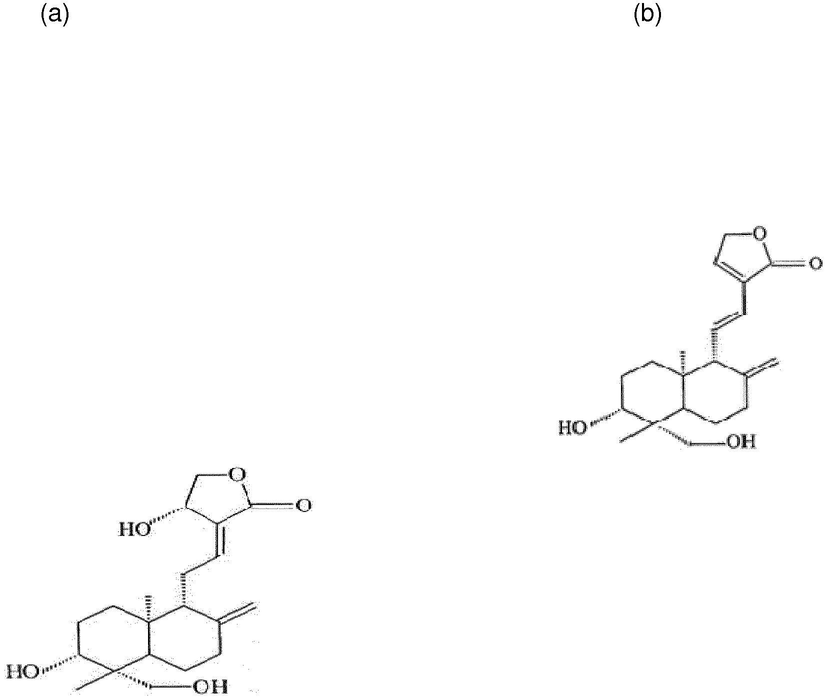

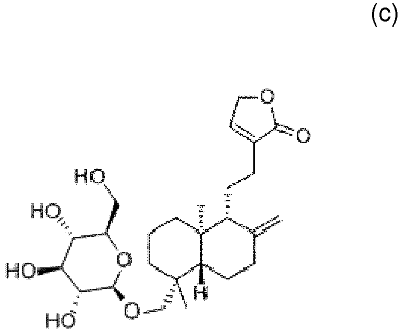

In certain embodiments the diterpenoid lactone is andrographolide, an unsaturated trihydroxy lactone having molecular formula of C₂₀H₃₀O₅.

The plant diterpenoid lactones of the composition of the invention may be obtained by conventional solid-liquid extraction of the bioactive components, andrographolide especially, from a vegetal part of a plant containing one or more of them, such as *Andrographis paniculata.*

In the field of plant extraction, methods based on extraction techniques are well known for obtaining the above bioactive diterpenoid lactones and separating them from natural sources.

In general, suitable techniques for extracting the bioactive ingredients (a), (b) and (c) from plant portion, in particular leaves, are those described hereinabove for extracting parthenolide from a plant part.

Suitable solvents include organic polar solvent, in particular ethanol.

Soxhlet extraction is a suitable technique for extracting and separating the bioactive terpenoids of formulae (a), (b) or (c) from natural sources.

In general the vegetal sample from Andrographis paniculata may be brought into contact with one or more portion of a polar organic solvent of the types mentioned above, optionally raising the temperature of the system to a range of 30 to 70°C such that the heat applied to the distillation flask reaches the extraction cavity. Usually no filtration is needed after the leaching step. In certain embodiments sample throughput can be increased by simultaneous extraction in parallel.

A suitable solid-liquid extraction of plant terpenoids in particular andrographolide is disclosed by R. Wongkittipong et al. in Solid-liquid extraction of Andrographolide from Plants-Experimental Study, Kinetic Reaction and Model; Sepn. Purifn. Technol. 40:147-154. In certain embodiments, the above terpenoid or a vegetal extract, portion or tissue of a plant containing such plant terpenoid may be present in the composition of the invention in an amount of 0,001 to 35% by weight, 0,01 to 20% by weight or 0,1 to 10% by weight.

The inventors have found that a mixture of parthenolide with one or more of the above plant diterpenoid lactone, as such or in the form of herbal extracts, attenuates hyperalgesia, reduces inflammation in neuronal context, inhibits the pro-inflammatory nuclear factor kappa B (Nf-kb) pathway, and the activation of anti-inflammatory Nrf2 pathway.

By means of such actions, the composition of the invention is effective in preventing and treating acute and recurrent neuropathic pain and migraine attacks.

In general, the association of an extract from *Tanacetum parthenium* and an extract from *Andrographispaniculata* unexpectedly inhibits Nf-kb activation and pro-inflammatory cytokines expression, and induce Nrf2 translocation and its related anti-inflammatory genes, in a cellular model of inflammation. Furthermore the mixture of the above extracts have shown the ability to effectively reduce hyperalgesia and neuropathic pain, in a well-established *in vivo* model of migraine. The inventors surprisingly found that association of parthenolide with at least one of plant terpenoids selected from andrographolide, neo-andrographolide, deoxyandrographolide, results in a synergistic activity against migraine and neuropathic pain.

In addition the association of bioactive ingredients of the composition of the invention reduces the incidence of recurrent migraine.

In general, with the terms bioactive ingredients or components of the composition are designed the above mentioned sesquiterpene lactone and/or the diterpenoid lactones.

The applicant has surprisingly found scientific evidence of synergism of the bioactive components of the composition of the invention with respect to specific measurable biological parameters, both at the cell and organism level.

In the present contest, with the term of "synergism or synergistic activity" is meant an activity which is greater than the sum of the activities of the bioactive ingredient when taken alone. Specifically, synergism occurs when at least two bioactive components interact in ways that enhance or magnify one or more effects, or side effects, of them. Thus, two bioactive components or drugs that produce overtly similar effects will sometimes produce exaggerated or diminished effects when used concurrently, and a quantitative assessment is necessary to distinguish these cases from simply additive action (Tallarida RJ. Drug synergism: its detection and applications. J PharmacolExpTher. 2001 Sep;298(3):865-72).

The biological effects on which the inventors focused are: 1) inhibition of nfkb activation and consequent reduction of pro-inflammatory cytokines induction; 2) positive modulation of nrf2 and consequent induction of antioxidant an anti-inflammatory genes.

The pharmacological definition of synergism, when translated in terms of molecular events, relates often to the ability of two bioactive substances producing synergism to enhance cell functions by eliciting pathways which are originally distinct but converge at the higher levels in elementary cell activities concurring to the same major cell program. In other words, if there are two ways for a cell to avoid death controlled by two unrelated or minimally related pathways A and B, enhancement of only A or B by two bioactive substances is likely to produce at the highest doses saturation of the pathway, and therefore a combined effect which is less than additive. Full stimulation of A and B, instead, could produce additivity or even synergism (a) because all the activities bringing to that response are engaged, (b) because the cross-talks taking place where the pathways converge can become mutually enhancing, or, finally, (c) because the synchronous activation of both allow the cell to move into discrete but sequential steps of activity.

In accordance with certain aspects the present invention provides a composition for use in the prophylaxis or treatment of migraine or neuropatic pain comprising a synergistic combination of a dry extract from Tanacetum partenium and a dry extract from Andrographis paniculata.

The model used by the inventors has been designed to prove that a *Tanacetum parthenium* extract, titled in pertenolides, and an *Andrographis paniculata* extract, titled in adrographolides, or a combination/mixture of parthenolide and a plant terpenoid selected from andrographolide, neo-andrographolide and deoxyandrographolide, have a synergistic activity in preventing migraine attacks, and more generally, neuropathic pain, even because each of them activates a pathway of cell response to inflammation and facilitate modulation of anti-inflammatory genes. More in detail, they have, in a synergistic way, a specific ability to inhibit Nf-kb activity and reduce cytokines expression, and the ability to induce Nrf2 activity and over express intracellular levels of anti-inflammatory and antioxidant genes.

Furthermore the two plant extracts of the composition are synergistic in reducing hyperalgesia and pain symptomatology, induced by formalin test (a well-established rat model of persistent somatic pain) followed by nitroglycerine administration (extensively used to investigate the neurobiological correlates of migraine pain) in rodents.

A *Tanacetum parthenium* extract and an *Andrographis paniculata* extract when combined for example in a mixture, have shown the ability to effectively reduce hyperalgesia and neuropathic pain, in a well-established *in vivo* model of migraine. By means of such action, the formulation object of the present application is effective in decreasing the incidence of recurrent migraine, preventing migraine attacks, and in general in the reduction of neuropathic pain.

Typically, the composition of the invention exerts a preventive effect, on the middle/long term of systematic intake, in retarding, preventing and partially treating migraine.

Furthermore the composition according to the present invention is for use for reducing the muscular tension.

In certain embodiments one or more of the above disclosed active ingredients of the composition of the invention are provided in the form of dry vegetal extracts.

In certain embodiments the composition of the invention further comprises additional active ingredients.

In certain embodiments the composition of the invention further comprises an additional bioactive ingredients selected from riboflavin, magnesium, Co-Q10 and mixtures thereof.

In certain embodiments the composition of the invention further includes one or more vitamins, such as vitamin of the B group, niacin, vitamin A, vitamin C, vitamin PP and others.

In accordance with some embodiments the composition of the invention also includes one or micro micronutrients and/or minerals or micronutrients such as salts of Mg, K, Na, Zn, Fe, Cr, Se, Mn, and others.

The term "carrier" as used herein refers to a vehicle, excipient, diluent, or adjuvant with which the association of therapeutic or active ingredients is administered. Any carrier and/or excipient suitable for the form of preparation desired for administration to human beings is contemplated for use with the compounds disclosed herein.

For purposes of the present application, the term "edible" is intended to mean food grade materials which are approved by regulatory authorities for use in pharmaceutical or food applications.

Typically, a suitable edible carrier is a physiologically or pharmaceutically acceptable carrier.

The compositions of the present invention encompass any compositions made by admixing the association of active ingredients of vegetal origins of the present invention and a pharmaceutically acceptable carrier. Such compositions are suitable for nutritional, pharmaceutical or dietetic use in a mammal, specifically human beings.

The carrier may take a wide variety of forms depending on the form of preparation desired for the oral administration, e.g., tablets, capsules, powders. In preparing the compositions for oral dosage form, any of the usual nutritional or pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

In certain embodiments, the extracts or bioactive ingredients contained in the composition of the present invention can be combined as the active ingredients in intimate admixture with a suitable edible carrier and/or excipient according to conventional pharmaceutical/nutritional techniques or food processing.

An exemplary route of administration of the composition of the invention is the oral route.

The compositions may be conveniently presented in unit dosage form and prepared by any of the methods well-known in the art of pharmacy or food processing.

The pharmaceutical compositions may be in the form of tablets, pills, capsules, solutions, suspensions, emulsion, powders, and as sustained release formulations.

If desired, tablets may be coated by conventional techniques.

In certain embodiments such compositions or preparations of the invention can contain at least 0.1 percent of each of the bioactive ingredients/substances. The percentage of active ingredient in these compositions may, of course, be varied and may conveniently be between about 1 percent to about 60 percent of the weight of the unit. The amount of active compound in such compositions is such that a prophylactic or therapeutically effective dosage will be obtained.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

In certain embodiments, the composition of the invention further includes one or more additional components, such as additives, fillers, stabilizers, emulsifying, texturizing, filmogenic, plasticizing, wetting agents, and thickeners.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor. To prevent breakdown during transit through the upper portion of the gastrointestinal tract, the composition may be an enteric coated formulation.

In some embodiments, in the pharmaceutical compositions of the present invention the bioactive ingredients are generally formulated in dosage units. The dosage unit may contain from 0.1 to 1000 mg of active ingredient or plant extract per dosage unit for daily administration.

In some embodiments, the amounts effective for formulation will depend on the severity of the disease, disorder or condition, previous therapy, the individual's health status and response to the association of active ingredients. In some embodiments, the dose is in the range from 0.001% by weight to about 60% by weight of the formulation.

In accordance with certain embodiments the composition of the invention is a herbal drugs that is product containing plant materials or plant parts as active ingredients in the crude or processed state and ore or more carriers.

In accordance with certain embodiments the composition of the invention is a supplement or nutritional product.

The following examples are provided merely for illustrating the present invention.

### Example 1

Synergistic composition for preventing or treating migraine of the following formulation:

| Active ingredient | Dosage range |
|---|---|
| Andrographis Paniculata (dry extract) | 50-500 mg |
| Tanacetum Parthenium (dry extract) | 50-500 mg |

### Example 2

A composition for the prevention and treatment of migraine having the following formulation:

| Active ingredient: | Dosage range: |
|---|---|
| Andrographis Paniculata (dry extract) | 50-500 mg |
| Tanacetum Parthenium (dry extract) | 50-500 mg |
| Magnesium (hydroxide) | 100-600 mg |
| Coenzyme Q10 | 5-400 mg |
| Riboflavin | 5-800 mg |

### Example 3

### Experimental evidence

### Effect of a composition containing parthenolide and andrographolide on inflammatory cytokines secretion in cultured microglia.

Microglia, one of the glial cell types in the CNS, is an important integral component of the neuro-glial cell network. There is accumulating evidence that microglia are involved in a variety of physiological and pathological processes in the brain by interacting with neurons and other glial cells and through production of biologically active substances such as growth factors, cytokines, and other factors. The role of inflammation in the activation of microglia as a potential causative agent of migraine attacks has been reconsidered recently, and drugs that block glial cell activation and the subsequent release of neuroexcitatory/neuroinflammatory substances might have therapeutic potential in the treatment of acute migraine and its prevention.

Primary rat's microglia from ScienCell Research Laboratories were used. Cells were pretreated or not for 1 h with parthenolide 5 µm, andrographolide 5 µm or parthenolide+ andrographolide 5 µm, and then exposed to 500 ng/ml LPS for another 24 h. IL-1b, IL-6 and TNF-a were detected by ELISA kits purchased from Abcam, Cambridge, (MA) USA. Data are expressed as mean ±SEM, n=4; *p<0.001 vs. LPS, **p<0.001 vs. parthenolide or andrographolide alone.

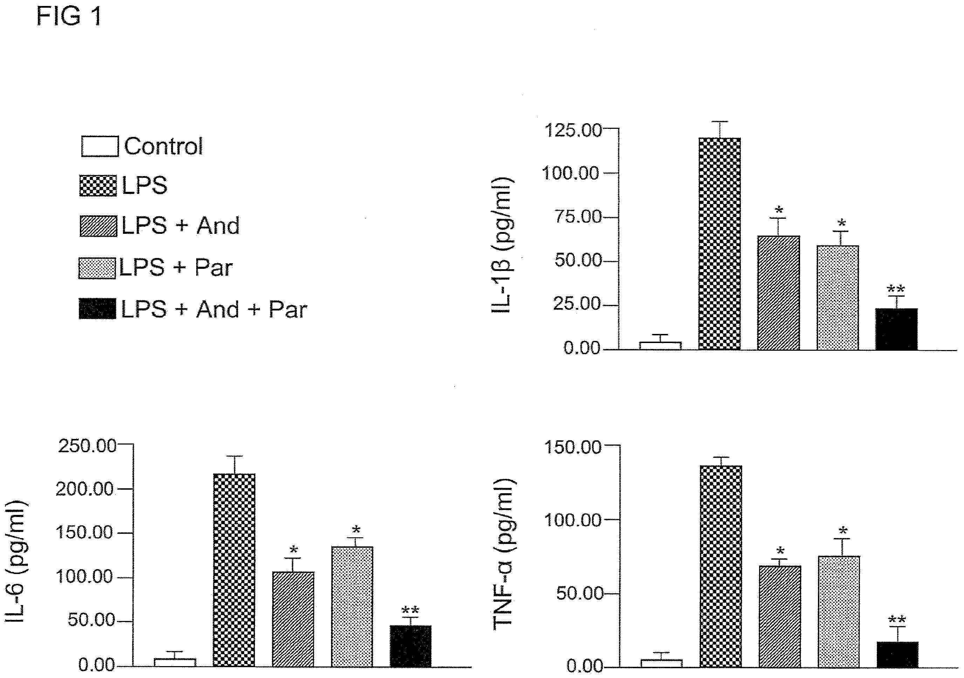

First, 24-h exposure of microglial cells to 500 ng/mL LPS induced the maximal secretion of IL-1β, IL-6 and TNF-α, which are all released in small amounts in basal conditions, as shown in Fig. 1.

Lipopolysaccharide strongly increased cytokines secretion. Preincubation (1 h) with parthenolide 5 µm or andrographolide 5 µm significantly reduced LPS-stimulated cytokines secretion. Surprisingly after 1-h incubation with a composition with the combination (mixture) of parthenolide + andrographolide dramatically reduced the secretion of IL-1β, IL-6 and TNF-α, demonstrating a synergistic effect of the two compounds in the inhibition of cytokines production.

### Example 4

### Experimental evidence

### Parthenolide and Andrographolide reduce NF-kB p65 DNA binding activity in cultured microglia

Many studies found that the NF-kB pathway play a pivotal role in the ethiogenesis of migraine attacks. The NF-kB family of transcriptional factors plays a critical role on regulation the expression of a wide variety of genes, particularly in the inflammatory process, included cytokines such as IL-1β, IL-6 and TNF-α.

To evaluate the ability of the synergistic composition of the invention to inhibit nf-kb activity in rats microglial cells we have used the NF-kB p65 DNA Binging Activity Assay by TransAM p65 transcription factor ELISA kit (Active Motif, Carlsbad, CA). Cells were stimulated with 500 ng/ml LPS in the presence and absence of parthenolide 5 µm, andrographolide 5 µm or a combination of parthenolide + andrographolide 5 µm, for 12 hours, according to the invention. Then, DNA binding activity of NF-kB p65 was determined.

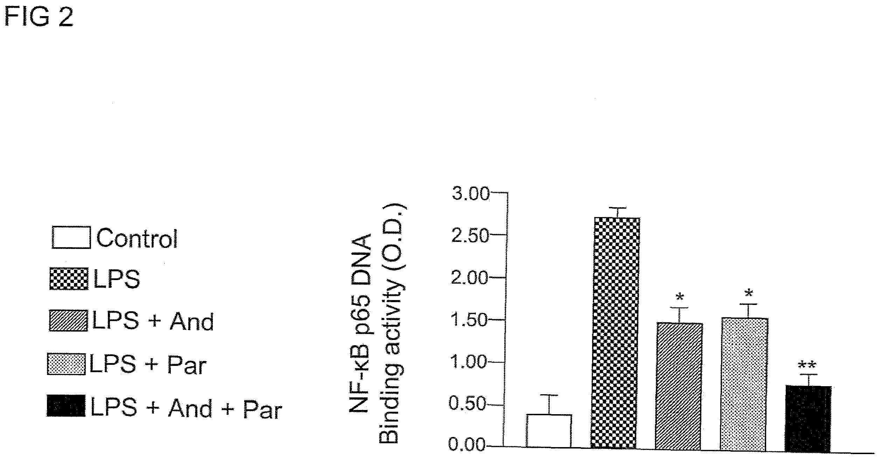

Each bar represents the mean ± SD (n = 4). *p<0.001 vs. LPS, **p<0.001 vs. parthenolide or andrographolide alone. 12 hours after LPS stimulation markedly promoted NF-kB p65 DNA binding activity. However, a lower DNA binding activity was observed in microglial cells with LPS stimulation in the presence of both parthenolide (PAR) and andrographolide (AND), as shown in Fig. 2. Surprisingly the combination of PAR +AND strongly reduced NF-kB p65 DNA binding activity, demonstrating a synergistic effect achieved by the combination of the two compounds.

### Example 5

### Experimental evidence

### Activation of Nrf2 expression in astrocytes by parthenolide or/and andrographolide

Cultured astrocytes were exposed to Parthenolide (PAR), Andrographolide (AND) or a combination of the two compounds, according to the invention, at the final concentration of 5 µM to evaluate the expression of Nrf2 protein over time.

Rat type 1 astrocytes (DI TNC1) were purchased from the American Type Culture Collection (Manassas, VA, USA) and cultured according to the manufacturer's instructions. Cells were grown in 75-cm2 flasks and maintained at 37°C in a humid atmosphere of air and 5% CO2.

Confluent cells were exposed to Parthenolide, Andrographolide or a combination of the two compounds at the final concentration of 5 µM, for different times (0, 1, 3, 6 and 12 hs). Astrocytes were washed twice with PBS 1X. Cells were then harvested in 1 ml PBS 1X and centrifuged at 3000 rpm for 3 min at 4°C. The cell pellet was carefully resuspended in 200 µl of cold buffer containing 10 m*M* HEPES (pH 7.9), 10 mM KCI, 0.1 mM EDTA, 0.1 mM EGTA, 1 µ*M* DTT, and complete protease inhibitor cocktail (Roche, Mannheim, Germany). The pellet was then incubated on ice for 15 min to allow cells to swell. After this time, 15 µl of 10% NP-40 was added and the tube was vortexed for 10 s. The homogenate was then centrifuged at 3000 rpm for 3 min at 4ºC and the nuclear pellet was resuspended in 30 µl of cold buffer consisting of 20 mM HEPES (pH 7.9), 0.4 *M* NaCl, 1 mM EDTA, 1 mM EGTA, 1 µ*M* DTT, and protease inhibitors. The pellet was then incubated on ice for 15 min and vortexed for 10-15 s every 2 min. The nuclear extract was finally centrifuged at 13,000 rpm for 5 min at 4ºC. The supernatant containing the nuclear proteins was resolved by SDS-polyacrylamide gel and submitted to immunoblot analysis using anti-Nrf2 (1:500 dilution) and anti-Sp1 (1:500 dilution) antibodies.

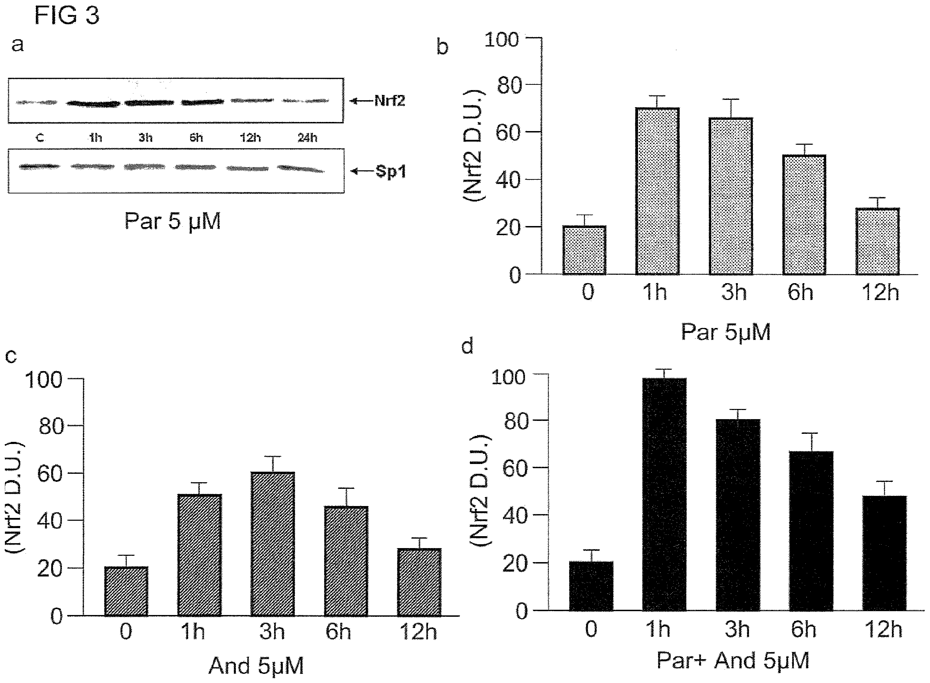

As shown in Figure 3, treatment with parthenolide caused a significant time-dependent increase in Nrf2 protein expression in the nuclear extracts. Quantification of three independent western blots showed that after 1h exposure to 5 µM Par, Nrf2 expression significantly increased and remained upregulated till 12h, whereas the levels of the housekeeping transcription factor Sp1 were stable. Andrographolide induced a lower increment of nuclear Nrf2. The combination of the two compounds induced in a massive way Nrf2 expression.

The experiment demonstrates that a combination of PAR and AND strongly activate Nrf2 pathway and consequently express defensive and reparative genes, such as the detoxifying enzymes of type II. Furthermore the tests show a synergistic effect of the combination of the two compounds (PAR and AND) in the ability to activate this signaling pathway.

### Example 6

### Experimental evidence

### Rats behavioral response to formalin test in basal condition and after nitroglycerin administration.

The formalin test is a well-established rat model of persistent somatic pain. Following injection of 100 II of 1% formalin into the plantar surface of the right hind paw, the animals were placed, one at a time, in a Plexiglas observation chamber (10 9 20 9 24 cm) in which a mirror (angled at 45) allowed unimpeded observation of the animal's paws. The total number of flinches/shakes per min was counted during the period from 1 to 5 min after injection (phase 1) and, subsequently, for 1-min periods at 5-min intervals during the period from 15 to 60 min (phase 2) after formalin injection. Flinches/shakes, characterized as a rapid and brief withdrawal or flexion of the injected paw, were readily identified. Incomplete formalin injection constituted an exclusion criterion for the study. The analgesic effect of parthenolide and andrographolide was evaluated by comparing the response to the formalin test of rats treated with phytochemicals versus untreated rats, in basal conditions and following nitroglycerin (NTG) administration (4h after the administration).

Systemic administration of NTG, a NO donor, provokes spontaneous-like migraine attacks in migraine sufferers. NTG also induces a condition of hyperalgesia in the rat, through the activation of spinal and brainstem structures involved in nociception; As such, NTG has been extensively used to investigate the neurobiological correlates of migraine pain, in rodents. Adult male Sprague-Dawley rats, weighing 180-220 g, were used in the present investigation. Rats were randomly divided in groups formed by 4-6 animals each, and underwent the following experimental protocols. Nitroglycerin (Astra Company, Italy), dissolved in saline alcohol and propylene glycol, was injected i.p. at a dose of 10 mg/kg. Experimental groups
- Control : i. p. injection of saline 4 h before the formalin test;
- Parthenolide (PAR): i. p. injection of saline suspension of Tanacetum parthenium extract (titled in parthenolide) 50 mg/kg, 4 h before the formalin test;
- Andrographolide (AND): i. p. injection of saline suspension of Andrographis panniculata extract (titled in andrographolides) 50 mg/kg, 4 h before the formalin test;
- PAR+AND: i.p. injection of saline suspension of the two compounds 25 mg/kg PAR+ 25 mg/kg AND, 4 h before the formalin test;
- NTG: i.p. injection of NTG (10 mg/kg) 4 h before the formalin test;
- PAR/NTG: i.p. injection of NTG (10 mg/kg) and PAR (50 mg/kg) 4 h before the formalin test
- AND/NTG: i.p. injection of NTG (10 mg/kg) and AND (50 mg/kg) 4 h before the formalin test
- PAR+AND/NTG: i.p. injection of NTG (10 mg/kg) and PAR (25 mg/kg) + AND (25 mg/kg) 4 h before the formalin test

In the formalin test, the total number of flinches/shakes evoked by formalin injection was counted in phases I and II of the formalin test, as described above. Differences between groups were analyzed by Student's t test and a probability level of<5% was regarded as valuable.

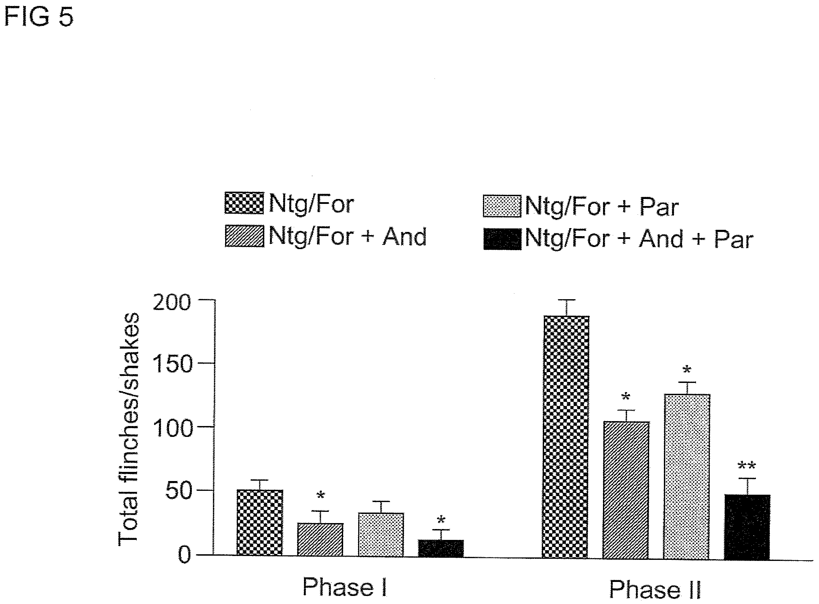

The injection of formalin resulted in a highly reliable, typical, biphasic pattern of flinches/shakes of the injected paw, being characterized by an initial acute phase of nociception within the first 5 min, followed by a prolonged tonic response from 15 to 60 min after formalin injection. Both parthenolides and andrographolides administration reduced the nociceptive behavior in both phases of the formalin test as shown in Fig. 5.

Surprisingly the combination of And + Par showed the the strongest activity. *p<0.05 versus For group. ** p<0.05 versus For+And and For+Par. Data were expressed as mean ± SD

NTG administration, 4 hours before the formalin test, significantly increased the total number of flinches/shakes in phase II of formalin test. Both parthenolides and andrographolides pre-treatment effectively inhibited the nociceptive behavior induced by NTG administration during phase II of the test (Fig. 6). The combination of And + Par again showed the strongest activity. *p<0.05 versus Ntg/For group. ** p<0.05 versus Ntg/For+And and Ntg/For+Par. Data were expressed as mean ± SD

### Example 7

A tablet for the prophylaxis of migraine and for reducing muscular tension having the following composition

| | |
|---|---|
| Partenio (Tanacetum partenium) dry extract | 150 mg |
| Andrographis paniculata dry extract | 100 mg |
| Magnesium | 281,25 mg |
| Coenzyme Q | 20 mg |
| Vitamin B 2 | 4,8mg |

## Claims

1. A composition for use in the prophylaxis and/or treatment of migraine and or neuropathic pain comprising a sesquiterpene lactone and a diterpenoid lactone in an effective amount and an edible carrier wherein the sesquiterpene lactone is parthenolide and the diterpenoid lactone is selected from andrographolide, neo-andrographolide, deoxyandrographolide and mixtures thereof.

2. A composition for use according to claim 1 wherein the sesquiterpene lactone parthenolide is contained in a vegetal extract from *Tanacetum parthenium.*

3. A composition for use according to claim 1 or 2 wherein sesquiterpene lactone parthenolide is in the form of a vegetal extract.

4. A composition for use according to anyone of claims 1-3 wherein the diterpenoid lactone is contained in a vegetal extract from *Andrographis paniculata.*

5. A composition for use according to claim 3 or 4 wherein the diterpenoid lactone is in the form of a vegetal extract.

6. A composition for use according to anyone of claims 1 to 5 wherein the diterpenoid lactone is andrographolide.

7. A composition for use according to anyone of claims 1 to 6 further comprising an additional active ingredients selected from riboflavin, magnesium, Co-Q10 and mixtures thereof.

8. A composition for use according to anyone of claims 1 to 7 in the form of a tablet or of a powder optionally encapsulated.

9. A composition for use according to anyone of claims 1 to 8 in the form of a phytomedicine, supplement, nutraceutical or herbal drug.

10. A composition for use according to claim 1 comprising a synergistic combination of a dry extract from Tanacetum partenium and a dry extract from Andrographis paniculata.

## Patentansprüche

1. Zusammensetzung zur Verwendung bei der Prophylaxe und/oder Behandlung von Migräne und/oder neuropathischem Schmerz, umfassend ein Sesquiterpenlacton und ein Diterpenoidlakton in einer wirksamen Menge und einen essbaren Träger, wobei das Sesquiterpenlacton Parthenolid ist und das Diterpenoidlacton ausgewählt ist aus Andrographolid, Neoandrographolid, Desoxyandrographolid und Mischungen davon.

2. Zusammensetzung zur Verwendung nach Anspruch 1, wobei das Sesquiterpenlactonparthenolid in einem pflanzlichen Extrakt aus *Tanacetum parthenium* enthalten ist.

3. Zusammensetzung zur Verwendung nach Anspruch 1 oder 2, wobei Sesquiterpenlactonparthenolid in Form eines Pflanzenextrakts vorliegt.

4. Zusammensetzung zur Verwendung nach einem der Ansprüche 1 bis 3, wobei das Diterpenoidlacton in einem pflanzlichen Extrakt von *Andrographis paniculata* enthalten ist.

5. Zusammensetzung zur Verwendung nach Anspruch 3 oder 4, wobei das Diterpenoidlacton in Form eines Pflanzenextrakts vorliegt.

6. Zusammensetzung zur Verwendung nach einem der Ansprüche 1 bis 5, wobei das Diterpenoidlacton Andrographolid ist.

7. Zusammensetzung zur Verwendung nach einem der Ansprüche 1 bis 6, weiterhin umfassend einen zusätzlichen Wirkstoff ausgewählt aus Riboflavin, Magnesium, Co-Q10 und Mischungen davon.

8. Zusammensetzung zur Verwendung nach einem der Ansprüche 1 bis 7 in Form einer Tablette oder eines optional eingekapselten Pulvers.

9. Zusammensetzung zur Verwendung nach einem der Ansprüche 1 bis 8 in Form eines phytomedizinischen, ergänzenden, nutrazeutischen oder pflanzlichen Arzneimittels.

10. Zusammensetzung zur Verwendung nach Anspruch 1, umfassend eine synergistische Kombination eines Trockenextrakts aus Tanacetum partenium und eines Trockenextrakts aus Andrographis paniculata.

## Revendications

1. Composition pour une utilisation dans la prophylaxie et/ou le traitement d'une migraine et/ou d'une douleur neuropathique, comprenant une lactone sesquiterpénique et une lactone diterpénoïde en une quantité efficace et un véhicule comestible, dans laquelle la lactone sesquiterpénique est le parthénolide et la lactone diterpénoïde est choisie parmi l'andrographolide, le néo-andrographolide, le désoxyandrographolide, et leurs mélanges.

2. Composition pour une utilisation selon la revendication 1, dans laquelle la lactone sesquiterpénique parthénolide est contenue dans un extrait végétal issu de *Tanacetum parthenium.*

3. Composition pour une utilisation selon la revendication 1 ou 2, dans laquelle la lactone sesquiterpénique parthénolide est sous la forme d'un extrait végétal.

4. Composition pour une utilisation selon l'une quelconque des revendications 1 à 3, dans laquelle la lactone diterpénoïde est contenue dans un extrait végétal issu *d'Andrographis paniculata.*

5. Composition pour une utilisation selon la revendication 3 ou 4, dans laquelle la lactone diterpénoïde est sous la forme d'un extrait végétal.

6. Composition pour une utilisation selon l'une quelconque des revendications 1 à 5, dans laquelle la lactone diterpénoïde est l'andrographolide.

7. Composition pour une utilisation selon l'une quelconque des revendications 1 à 6, comprenant en outre un agent actif additionnel choisi parmi la riboflavine, le magnésium, le Co-Q10 et leurs mélanges.

8. Composition pour une utilisation selon l'une quelconque des revendications 1 à 7, sous la forme d'un comprimé ou d'une poudre éventuellement encapsulée.

9. Composition pour une utilisation selon l'une quelconque des revendications 1 à 8, sous la forme d'un phytomédicament, d'un complément, d'un nutraceutique ou d'un médicament à base de plantes.

10. Composition pour une utilisation selon la revendication 1, comprenant une combinaison synergique d'un extrait sec de Tanacetum partenium et d'un extrait sec d'Andrographis paniculata.
